(19)

Europäisches
Patentamt
European
Patent Office
Office européen
des brevets

(11)  **EP 2 818 561 A1**

(12)  **EUROPEAN PATENT APPLICATION**

(43) Date of publication:
**31.12.2014  Bulletin 2015/01**

(51) Int Cl.:
**C12R 1/38** (2006.01)         **C12P 7/62** (2006.01)
**C12N 9/10** (2006.01)

(21) Application number: **13173572.2**

(22) Date of filing: **25.06.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Dritte Patentportfolio
Beteiligungsgesellschaft
mbH & Co. KG
12529 Schönefeld/OT Waltersdorf (DE)**

(72) Inventors:
• **Bassas Galià, Monica
1950 Sion (CH)**

• **Arias Rivas, Sagrario
38100 Braunschweig 1 (DE)**
• **Molinari, Gabriella
38304 Wolfenbüttel (DE)**
• **Timmis, Kenneth Nigel
38124 Braunschweig (DE)**

(74) Representative: **Held, Stephan
Meissner, Bolte & Partner GbR
Widenmayerstraße 47
80538 München (DE)**

(54) **Utilization of the novel, environmental isolates Pseudomonas sp. IPB-B26 and N-128 for the efficient high yield production of mcl/lcl-PHAs**

(57)   The present application is directed at microorganisms of the genus *Pseudomonas* as deposited under DSM26199 (*Pseudomonas sp.* IPB-B26) and DSM26200 (*Pseudomonas sp.* N-128) with the Leibnitz Institute DSMZ. The present application is further directed at a process for the production of medium- and long-chain-length PHAs, comprising cultivating said microorganisms in a culture medium comprising a carbon source and isolating the PHA from the microorganisms. It has been observed that these microorganisms allow for efficient PHA production in high yields. In addition, the inventive microorganisms possess the valuable capability to incorporate unsaturated fatty acids into the resulting PHAs. The inventive microorganisms thus enable a later modification of the PHAs as well as crosslinking, thus opening new fields of applications for these materials.

EP 2 818 561 A1

**Description**

The present invention is in the field of biosynthesis of polyhydroxyalkanoates (PHAs). The invention relates to wild type microorganisms of the genus *Pseudomonas* as deposited under DSM26199 (*Pseudomonas sp.* IPB-B26) and DSM26200 (*Pseudomonas sp.* N-128) with the Leibnitz Institute DSMZ German Collection of Microorganisms. These microorganisms have been proven to be of great utility in processes for the production of PHA. The microorganisms are non-genetically modified and have been observed to be capable to very efficiently producing medium- (mcl) /long-chain-length (lcl)-PHAs from various, rather inexpensive and sustainable feedstock like saturated and unsaturated fatty acids as well as glycerol. In the bioreactor, the microorganisms reach high biomass and PHA production, even under conditions of moderate stirring and without extra oxygen supply. Depending on the actual substrate used, the resulting PHAs may comprise unsaturated moieties (up to 17% and more), allowing for a much larger spectrum of PHA properties and/or post-synthetic functionalisation of the PHAs. The present invention is also directed to the use of these microorganisms in a process for the production of mcl- and/or lcl-PHAs, as well as to PHAs obtainable by such process.

**Background of the invention**

PHAs are polymers that are biodegradable and biocompatible thermoplastic materials (polyesters of 3-hydroxy fatty acids) produced from renewable resources with a broad range of industrial and biomedical applications (Williams & Peoples, 1996, Chemtech 26: 38-44). PHAs are synthesized by a broad range of bacteria and have been extensively studied due to their potential use to substitute conventional petrochemical-based plastics to protect the environment from harmful effects of plastic wastes.

PHAs can be divided into two groups according to the length of their side chains and their biosynthetic pathways. Those with short side chains, such as PHB, a homopolymer of (R)-3-hydroxybutyric acid units, are crystalline thermoplastics, whereas PHAs with long side chains are more elastomeric. The former have been known for about ninety years (Lemoigne & Roukhelman, 1925, Ann. Des Fermentation, 527-536), whereas the latter materials were discovered relatively recently (deSmet et al., 1983, J. Bacteriol. 154: 870-878). Before this designation, however, PHAs of microbial origin containing both (R)-3-hydroxybutyric acid units and longer side chain (R)-3-hydroxyacid units from 5 to 16 carbon atoms had been identified (Wallen & Rohweder, 1974, Environ. Sci. Technol. 8: 576-579). A number of bacteria which produce copolymers of (R)-3-hydroxybutyric acid and one or more long side chain hydroxyl acid units containing from 5 to 16 carbon atoms have been identified (Steinbuchel & Wiese, 1992, Appl. Microbiol. Biotechnol. 37: 691-697; Valentin et al., 1992, Appl. Microbiol. Biotechnol. 36: 507-514; Valentin et al., 1994, Appl. Microbiol. Biotechnol. 40: 710-716 ; Abe et al., 1994, Int. J. Biol. Macromol. 16: 115-119; Lee et al., 1995, Appl. Microbiol. Biotechnol. 42: 901-909; Kato et al., 1996, Appl. Microbiol. Biotechnol. 45: 363-370; Valentin et al., 1996, Appl. Microbiol. Biotechnol. 46: 261-267; US Patent No. 4,876,331). These copolymers can be referred to as PHB-co-HX (wherein X is a 3-hydroxyalkanoate or alkanoate or alkenoate of 6 or more carbons). A useful example of specific two-component copolymers is PHB-co-3-hydroxyhexanoate (PHB-co-3HH) (Brandl et al., 1989, Int. J. Biol. Macromol. 11: 49-55; Amos & McInerey, 1991, Arch. Microbiol. 155: 103-106; US Patent No. 5,292,860). Although PHAs have been extensively studied because of their potential use as renewable resources for biodegradable thermoplastics and biopolymers (as mentioned above) and have been commercially developed and marketed (Hrabak, 1992, FEMS Microbiol. Rev. 103: 251-256), their production costs are much higher than those of conventional petrochemical-based plastics, which represents a major obstacle to their wider use (Choi & Lee, 1997, Bioprocess Eng. 17: 335-342). As described above, many bacteria produce PHAs, e.g. *Alcaligenes eutrophus, Alcaligenes latus, Azotobacter vinlandii, Pseudomonas acitophila, Pseudomonas oleovarans, Eschericha coli, Rhodococcus eutropha, Chromobacterium violaceum, Chromatium vinosum, Alcanivorax borkumensis* etc. All PHA-producing bacteria known in the art produce intracellular PHA and accumulate it in PHA granules (Steinbüchel, 1991, Biomaterials, pp. 123-213). The main aspects, which render PHA production expensive and therefore unfavorable as compared to petrochemical-based plastics, are that it is difficult to produce the material in high yield and to recover the produced PHA from within the bacterial cells where it is accumulated. In order to reduce the total production costs of PHA, the development of an efficient recovery process was considered to be necessary generally aiming at cell disruption (Lee, 1996, Biotech. Bioeng. 49: 1-14) by i) an appropriate solvent, ii) hypochlorite extraction of PHA and/or iii) digestion of non-PHA cellular materials.

At an industrial scale, the available microorganisms still provide relatively little PHA, which renders the production of PHA with these microorganisms economically non-feasible. All methods known in the art require large amounts of water during the production and in addition chemical reagents and/or enzymes for their recovery, which is an obstacle to reducing the production costs. Therefore, alternative strategies for PHA production are in urgent need.

In the recent past, strategies for the genetic modification of PHA-producing microorganisms have been developed, e.g. to enable the microorganisms to produce higher amounts of PHA. EP 1 913 135 A1 describes microorganisms, which have been genetically modified by knocking-out genes, which act on intermediates for the PHA production in a competitive manner to PHA synthases. By depleting the microorganisms of enzymes, which interfere with PHA synthase for intermediates, it was possible to channel the intermediate's conversion towards PHA.

Another approach was to introduce PHA synthases into microorganisms such as *e.g. Escherichia coli,* which in their wild type form are not capable to produce PHA (cf. Qi et al., 2007, FEMS Microbiol. Lett. 157: 155-162). In this case, a maximum PHA accumulation of about 15% CDW (cell dry weight) was observed in an *E. coli* LS1298 strain, when decanoate was used as the carbon source.

In a yet alternative approach, the PHA production was increased by knock-out of the PHA depolymerase gene, which in the microorganism *P. putida* KT2440 led to yields of about 4 g/L CDW with PHA accounting for up to 80% of the CDW (Cai et al., 2009, Bioresource Techn. 100: 2265-2270).

Despite of these advancements, the amount of PHA produced by these microorganisms compared with the resources necessary for their production is still relatively low. In addition, in some countries there are public reservations against genetically engineered microorganisms in general, which leads to problems in terms of acceptance of these materials. In particular for these countries, it would be advantageous to have wild type, i.e. non-genetically modified microorganisms, which produce PHAs in high yields.

Most microorganisms, which have until now been described for PHA production, only accept saturated fatty acids as carbon sources for the production of PHAs. PHAs produced from regular substrates such as straight chain saturated fatty acids with a chain length of 6 to about 20 carbon atoms usually exhibit glass transition temperatures of the polymers in the range of -30°C to -50°C. This limits their utility to applications, which are compatible with such glass transition temperatures. If the scope of substrates accepted by corresponding microorganisms for incorporation into PHAs could be extended, this would have a great impact on the diversity of the properties of PHAs accessible from such microorganisms. In addition, if functional groups could be inserted into the PHAs, which allow for post-production modifications, this would have a great impact on the diversity of PHA products accessible from such microorganisms. For instance, unsaturated carbon-carbon double bonds present in the PHAs could provide a reactive center in the obtained material, which could be used for subsequent modifications and functionalisations such as attachment of conventional unsaturated monomers including acrylates and other vinylic monomers or crosslinking. The PHAs would thus allow for the manufacture of e.g. elastomeric materials or impact modifiers, wherein petrochemical-based plastics could at least be partially replaced by PHAs, which are produced in a biological process.

The present application addresses these needs.

Brief **description of** the invention

One aim of the present application is to provide non-genetically modified (i.e. wild type) microorganisms of the genus *Pseudomonas* as deposited under DSM26199 *(Pseudomonas sp.* IPB-B26) and DSM26200 *(Pseudomonas sp.* N-128) with the Leibnitz Institute DSMZ, Inhoffenstr. 7B, 38124 Braunschweig, Germany. The inventive microorganisms *Pseudomonas sp.* IPB-B26 and *Pseudomonas sp.* N-128 were isolated from an enrichment culture of different contaminated (with hydrocarbons, Diesel and petroleum) soils using crude oil (1%) and olive oil (1%) as substrates. In 500 ml-shake flasks, the strains have shown good biomass and PHA yields of up to 6 g/L and 2.4 g/L (about 40 wt.-% PHA accumulation), respectively, when grown with oleic acid as a substrate.

The present application is further directed to a process for the production of medium- and/or long-chain-length PHAs comprising

- cultivating a microorganism of the genus *Pseudomonas* as deposited under DSM26199 *(Pseudomonas sp.* IPB-B26) or DSM26200 *(Pseudomonas sp.* N-128) with the DSMZ in a suitable medium and in the presence of a carbon source and
- isolating the PHA from the respective microorganism.

Further aspects of the present application are directed at PHAs obtainable from said process, wherein the PHAs preferably comprise unsaturated moieties and the use of the above-mentioned microorganisms in a process for the production of mcl- and/or lcl-PHAs.

**Brief description of the drawings**

**Figure 1** Kinetic profiles of biomass and PHA production and ammonium consumption along the batch fermentation of *Pseudomonas sp.* IPB-B26 using oleic acid as substrate. Values are means of duplicates. At t = 100 min, upper curve:

CDW (in g/L); middle curve: PHA (in g/L); and lower curve: ammonium concentration (in mg/L).

**Figure 2** Feed rate for the oleic acid and MgSO$_4$ during the fed-batch fermentation.

**Figure 3** Oleic acid and ammonium consumption during the fed-batch fermentation. Oleic acid concentration present in the culture was determined by HPLC analysis.

**Figure 4** Growth, biomass and PHA production in the fed-batch fermentation using *Pseudomonas sp.* IPB-B26 and oleic acid as carbon source. Results are means of duplicates.

## Description of the invention

Medium-chain, as this term is used in the context of the present invention is intended to mean hydroxyl acid units ((R)-3-hydroxyacid units) with 5 to 13 carbon atoms. The term "long-chain-length PHA" is intended to encompass PHAs, containing at least 14 carbon atoms per monomer.

In the course of the inventor's investigations, it has been discovered that the medium used for the fermentation of the inventive microorganisms has a significant impact on the PHA productivity of the microorganisms. From several production media tested, MM medium modified with 0.1% yeast extract (as described in Martinez-Blanko et al., 1990, J. Biol. Chem. 265: 7084-7090) provided the lowest PHA productivity when oleic acid (1%) was used as the carbon source, and when *Pseudomonas sp.* IPB-B26 was used as the microorganism. Under the same conditions *Pseudomonas sp.* N-128 provided reasonable PHA yields. For the strain *Pseudomonas sp.* IPB-B26, the medium C-Y as described in Choi et al. (1994, Appl. Environ. Microbiol. 60: 1245-1254) provided significantly better yields, which could be even further increased when the amount of nitrogen in this medium was doubled. In the practice of the present application, the growth of *Pseudomonas sp.* IPB-B26 in C-Y medium is therefore preferred over the use of MM medium + 0.1% yeast extract. For *Pseudomonas sp.* N-128, the yields with C-Y-medium was lower compared to MM medium + 0.1% yeast extract, but the PHA yield recovered when the nitrogen concentration in C-Y-medium was doubled to provide comparable amounts of PHA. For this strain it is thus preferred, that if C-Y-medium is used in the fermentation, the medium should comprise a nitrogen content of about twice the amount indicated in Choi et al. For both *Pseudomonas* strains, N-128 and IPB-B26, it was observed that E2 medium (as described by Vogel & Borner (1956, J. Biol. Chem. 218: 97-106) provided the best results. With this medium, using 500 ml-flasks with 100 ml culture at 30°C and 200 rpm, PHA yields of about 2 g/Land cell dry weights (CDW) exceeding 5.1 g/Lwere obtained for both strains. In the practice of the present application, it is therefore preferred that the culture medium for the fermentation is E2 medium as described above.

The inventive process is not subject to any relevant restrictions as concerns the carbon source to be employed for the production of PHA. Carbon sources, which are regularly used for the production of PHAs, can be used with the microorganisms of the present application in the inventive process such as glycerol, sugars, pyruvate, and conventional fatty acids such as in particular fatty acids comprising 4 to 20 carbon atoms and preferably 8 to 18 fatty carbon atoms. It has been discovered, however, that the best yields of PHA in terms of g/L were obtained, if fatty acids are used as the carbon source. Consequently, a preferred process of the present application involves a carbon source, which comprises at least one C4 to C20 fatty acid, preferably a C8 to C18 fatty acid. The preferred saturated fatty acids for use in the present application are butyric acid, valeric acid, hexanoic acid, heptanoic acid, caprylic acid, nonanoic acid, decanoic acid, lauric acid, myristic acid, palmitic acid, heptadecanoic acid, stearic acid, and aracidic acid.

It has further been discovered, that the inventive microorganisms also accept unsaturated fatty acids such as oleic acid and 10-undecenoic acid as a substrate. A preferred embodiment of the inventive process thus involves fatty acids as carbon sources, which comprise one or more unsaturated moieties, preferably a single unsaturated moiety. Representative unsaturated fatty acids comprise myristoleic acid, palmitoleic acid, sapienic acid, oleic acid, elaidic acid, vaccenic acid, linoleic acid, linoelaidic acid, $\alpha$-linoleic acid, arachidonic acid, eicosapentaenoic acid, and undecenoic acid. The most preferred unsaturated fatty acid for use in the inventive process is oleic acid.

If the process of the present application is a shake-flash- or batch process, it is further preferred that the carbon to nitrogen (C/N) ratio in a culture medium is in the range of about 9:1 to 70:1, preferably in the range of about 15:1 to 50:1. If the (C/N) ratio is less than 9:1 or in excess of 70:1, the PHA yields of the resulting product were usually lower than in the preferred range.

In one embodiment of the present application, the carbon source is added in a single lump to the cultivation mixture at the start of the cultivation. It was observed in this regard that if the carbon source was added in e.g. two portions, one of which being added at the beginning of the cultivation and the second of which at a later stage, the PHA yield both in g/Land wt.-% was lower compared to a process wherein the carbon source has been added as a single lump.

In the context of a shake-flask or batch-process, it is further preferred that the amount of carbon source added to the cultivating mixture is such that a concentration of the carbon source in the cultivating mixture in a range of about 1 to 60 mM, preferably in the range of about 10 to 40 mM. If the carbon source is added to provide a concentration of less than 1 mM, the yield of PHA was lower than in fermentations wherein the concentration of the carbon source was in the indicated ranges. If the carbon source concentration is in excess of 60 mM, the environment becomes increasingly toxic to the cells, which negatively impacts their growth.

A further important parameter of the inventive process is the nitrogen content in the culture medium, as nitrogen is an important nutrient for the microorganisms, and PHA production is usually favoured under conditions, featuring an excess of carbon and a certain deficiency of e.g. nitrogen. In a preferred process of the present invention, an ammonium salt is used as the nitrogen source such as for example ammonium sulphate or ammonium hydroxide.

The ammonium concentration in the cultivation medium is further preferably in the range of about 10 to 60 mM, in particular in the range of about 15 to 40 mM. However, ultimately it is the C/N ratio, rather than the actual concentration of the nitrogen source, which has the largest impact on the strain's growth and PHA production.

A further important aspect of the present application is the oxygen concentration in the fermentation as the microorganisms consume oxygen to convert the carboxylic acids to 3-hydroxycarboxylic acids. In the practice of the present application, it is preferred that the partial pressure of oxygen ($pO_2$) is maintained between about 25% and 45%, preferably at about 30% in the cultivation medium, wherein % is %-mol and calculated based on the total gas dissolved in the cultivation medium.

With regard to the cultivation time, the present application is not subject to any relevant restrictions. The skilled practitioner will be aware, however, that during the cultivation, the amount of PHA produced at some stage will reach a maximum after which either the PHA-content declines or no longer changes. The skilled practitioner will be readily capable to determine the time wherein the amount of PHA accumulation in the microorganisms is highest. As a rule of a thumb, the maximum PHA accumulation in a fed-batch process was usually reached after about 40 hours and before about 100 hours. Therefore, the cultivation is preferably carried out for a time of not less than 40 h and not more than 96 h, preferably for not less than 45 h to not more than 60 h and most preferably about 48 h.

For the inventive microorganisms, a temperature of about 30°C has been determined as the optimum temperature for PHA production. Therefore, the process of the present application is preferably run at temperatures of from about 15°C to 45°C and preferably from about 20°C to 40°C.

In an embodiment of the present application, which is different to the above-mentioned batch-process, the carbon source is supplied to the cultivating medium in a fed-batch manner, i.e. a manner, which involves the supplementation of an exponentially increasing carbon dosage after an initialization time of the fermentation. The parameters from the calculation of the exponentially increasing carbon dosage was calculated based on the following equation:

$$F(t) = \mu \cdot \frac{V_0 \cdot X_0}{S_0 \cdot Yx/s} \cdot e^{-\mu set \cdot t}$$

wherein F(t) is the flow rate of the carbon source along the cultivation, $V_0$ is the volume of the culture, $Y_{x/s}$ is the yield of biomass, $X_0$ is the initial biomass after the batch culture, $\mu_{set}$ is the desired specific growth rate, and $S_0$ is substrate concentration in the feed.

$\mu_{set}$ in the inventive process is preferably in the range of about 0.05 to 0.15 h$^{-1}$, more preferably in the range of about 0.08 to 0.12 h$^{-1}$.

The above-mentioned fed-batch process allows for a substantial improvement of the yields of both, biomass and PHA, as well as reduction of the fermentation time to reach these maximum yields, wherein the optimum PHA concentration in the fermentation could be increased by a factor of 10 and reached after about 40 to 48 h. This represents significant advantages over the conventional batch process (see above).

In the afore-mentioned process, it is preferred that prior to the addition of an exponentially increasing carbon source dosage, the fermentation is initialized in a batch phase wherein an initial lump of carbon source is added to the cultivating medium and the culture is subsequently maintained for a time sufficient to ensure complete initial carbon source consumption. In the practice of the present invention it has been observed that the initial batch phase is suitably carried out for a time of from about 8 to 24 h, preferably for 8 to 12 h.

In the fed-batch process, it is further preferred that the initial lump of carbon source provides a carbon source concentration in the cultivating medium in the range of about 2 to 30 mM, preferably from about 5 to 15 mM. This range had been determined to provide optimal initial cultivation before onset of the exponential feeding process.

The stirring rate of the fermentation mixture in the batch or fed-batch process is not subject to any relevant limitations except that it has to be sufficient to maintain a partial pressure of oxygen ($pO_2$) in the above-indicated ranges. Suitable stirring rates depend on the requirements of the fermentation, but are usually within the range of about 200 to 1400 rpm.

The microorganisms of the present invention have unexpectedly been discovered to exhibit fusion of PHA granules to a single granule during the fermentation, while initially multiple PHA granules were formed.

As concerns the isolation of the PHA from the microorganisms, it is preferred that a PHA is extracted with a non-chlorinated solvent, preferably with a ketone having 3 to 8 carbon atoms. Non-chlorinated solvents provide the advantage of significantly lower waste disposal problems and costs compared to conventional chlorinated solvents such as chloroform and dichloromethane. The referred ketones for use in the practice of the present application are acetone, 2-methylethylketone, diethylketone, 2-methylpropylketone, etc. The most preferred ketone for use in the isolation of PHA is acetone.

It is further preferred, that the PHA is extracted at temperatures of less than about 60°C, preferably at temperatures of from about 20°C to 40°C. It has unexpectedly been discovered that the extraction of the inventive microorganisms at these temperatures provide substantially the same yields as comparable extractions at higher temperatures. It is believed that this is a direct result from the formation of a single PHA-granule and the disruption of microorganism cell walls observable toward the end of the fermentation process. Thus, in the inventive microorganisms the PHA is easier to access for the solvents than the multiple granules in a microorganism of a conventional fermentation. It had further been observed that substantially the same yield of extracted PHA could be obtained after extractions for about 0.5 to 5 h. It is thus preferred that the solvent extraction is carried for a time of about 1 to 3 hours, preferably for about 1 hour.

A further aspect of the present application concerns PHAs obtainable by the process as described above. Preferably, the process involves the incorporation of carboxylic acids, comprising up to 17%-mol or more of unsaturated moieties.

A yet further aspect of the present application is the use of a microorganism as described above in a process for the production of medium- or long-chain-length PHAs. Preferred embodiments of this process are identical to those described for the process for the production of medium- or long-chain-length PHAs above.

A final aspect of the present application is the use of a PHA synthase as deposited in the Gene Bank (NCBI) under the Accession number JN651420 (*phaC1*) or JN216885 (*phaC2*) or analogues thereof for the production of PHA. The PHA synthases or analogues thereof may be used either alone or in mixtures thereof. An "analogues" as this term is used in the practice of the present invention is indented to mean a peptide or protein, which has at least about 80% sequence identity, preferably at least about 90% sequence identity, more preferably at least about 95% sequence identity, and most preferably at least about 98% sequence identity, and has comparable properties in that it is capable to efficiently synthesize PHA under appropriate conditions.

In the following the present application will be described further by way of examples, which, however, are not intended to limit the scope of the present application by any means.

**ExampLe 1**

In order to select the best media for PHA production, both strains *Pseudomonas sp.* IPB-B26 and *Pseudomonas sp.* N-128, respectively, were cultivated in 500 ml-flasks with 100 ml of the respected medium, containing oleic acid (1%) as the carbon source, at 30°C with a stirring at 200 rpm. Cells were harvested for analysis after 72 h of culturing. The following media were tested:

1. E2 medium as described by Vogel & Borner, 1956, J. Biol. Chem. 218: 97-106.
2. MM medium + 0.1% yeast extract as described by Martinez-Blanko et al., 1990, J. Biol. Chem. 265: 7084-7090.
3. C-Y medium as described by Choi et al., 1994, Appl. Environ. Microbiol. 60: 3245-3254 with regular and twice the nitrogen concentration (0.66 und 1.32 g/L $(NH_4)_2SO_4$).

The results of these investigations are presented in the following Table 1.

Table 1 Biomass and PHA production of strains N-128 and IPB-B26 in different culture media.

| Strain (substrate) | Medium | CDW (g/L) | PHA (g/L) | PHA (%wt) |
|---|---|---|---|---|
| N-128 (oleic acid 1%) | E2 | 5.14 | 1.90 | 36.6 |
| | MM+0.1%YE | 5.51 | 1.69 | 29.7 |
| | C-Y | 2.27 | 0.94 | 42.2 |
| | C-Y (x2N) | 3.64 | 1.74 | 47.8 |

(continued)

| Strain (substrate) | Medium | CDW (g/L) | PHA (g/L) | PHA (%wt) |
|---|---|---|---|---|
| **IPB-B26 (oleic acid 1l)** | **E2** | 6.02 | 2.32 | 37.5 |
| | **MM+0.1%YE** | 4.44 | 0.70 | 15.8 |
| | **C-Y** | 3.91 | 2.16 | 53.9 |
| | **C-Y (x2N)** | 5.03 | 2.05 | 41.0 |
| Results were obtained after 72 h of incubation at 30°C and 200 rpm. Values are means of duplicates. | | | | |

Both strains grew in the different media. PHA production was lower for IPB-B26 if MM + 0.1% yeast extract was used as the fermentation medium. It is remarkable, that in C-Y media the strain IPB-B26 provided 54 wt.-% of PHA accumulation. Nevertheless, biomass production was lower for this medium than in the other two medias tested (E2 and MM + 0.1% yeast extract) forboth strains. After increasing the ammonium concentration in C-Y medium by two fold, it was possible to increase the biomass production up to 3.64 g/Land 5.03 g/Lwith a PHA accumulation of about 48 wt.-% and 41 wt.-% for *Pseudomonas spp.* N-128 and IPB-B26, respectively.

In general, media C-Y(2N) and E2 showed the highest yields of PHA production. Even though the PHA production was similar, biomass production was significantly higher in E2 medium. Accordingly, E2 is considered the preferred medium.

## Example 2

The cultivation of *Pseudomonas spp.* N-128 and IPB-B26 was repeated with E2 medium as described in Example 1, while the carbon source was changed from oleic acid (1%) to octanoic acid (20 mM), glycerol (3%) or crude glycerol (3%). The results of these investigations are presented in the following Table 2 below.

**Table 2** Biomass and PHA production of strains N-128 and IPB-B26 in E2 medium.

| Strain | Substrate | CDW (g/L) | PHA (g/L) | PHA (%wt) |
|---|---|---|---|---|
| **N-128** | **Oleic (1%)** | 5.14 | 1.90 | 36.6 |
| | **Octanoic (20 mM)** | 1.77 | 0.27 | 15.4 |
| | **Glycerol (3%)** | 3.00 | 0.50 | 16.0 |
| | **Crude glycerol (3%)** | 3.88 | 0.80 | 21.6 |
| **IPB-B26** | **Oleic (1%)** | 6.02 | 2.32 | 37.5 |
| | **Octanoic (20 mM)** | 1.76 | 0.14 | 8.0 |
| | **Glycerol (3%)** | 4.82 | 1.40 | 29.0 |
| | **Crude glycerol (3%)** | 4.46 | 1.40 | 30.5 |
| Values are means of triplicates. | | | | |

Even though glycerol and crude glycerol are good substrates for both strains, the PHA yields obtained with oleic acid were significantly higher.

## Example 3

The PHA polymers obtained using oleic acid and glycerol as a substrate were purified and analyzed by NMR and GCMS. The results of these investigations are presented in the following Table 3.

**Table 3** Monomer composition of the PHA-polymers produced by strains IPB-B26 and N-128

|  | PHA-N128 E2-oleic | PHA-B26 E2-oleic | PHA-B26 E2-glycerol |
|---|---|---|---|
| PHA (g/L) | 1.90 | 2.32 | 1.40 |
| **Monomer composition** | | | |
| 3OHC4 | 0.4 | 0.4 | 2.0 |
| 3OHC5 | | 2.0 | |
| 3OHC6 | 5.1 | 4.9 | 3.7 |
| 3OHC8 | 40.9 | 33.5 | 22.1 |
| 3OHC10 | 27.5 | 32.4 | 42.9 |
| 3OHC12:0 | 12.1 | 9.4 | 12.2 |
| 3OHC12:1 | | 0.7 | 14.7 |
| 3OHC14:0 | | 6.5 | |
| 3OHC14:1 | 14.0 | 10.2 | 2.3 |

Between the two PHAs obtained from oleic acid, the one produced by strain *Pseudomonas sp.* IPB-B26 shows a larger diversity in terms of monomer composition than the one obtained with *Pseudomonas sp.* N-128, containing monomers ranging from C4-C14 (number indicative for the number of carbon atoms). Surprising is the presence of 3-hydroxyvaleric acids (3OHC5), featuring an uneven number of carbon atoms. In general, both PHAs derived from oleic acid contained 30HC6 (about 5%-mol), 30HC8 (27-32%-mol), 3OHC10 (27-32%-mol), 30HC12 (9-12%-mol) and 3OHC14:1 (10-14%-mol) (wherein 14:1 indicates 14 total carbon atoms and 1 unsaturated double-bond).

The PHA derived from glycerol shows differences (compared to the PHAs obtained from oleic acid) especially in the content of the unsaturated monomers 3OHC12:1 and 3OHC14:1. The 3OHC8 (22.1%-mol) and 3OHC10 (42.9%-mol) are still the major monomeric units. On the other hand, there is an increase of the 3OHC12:1 monomer (up to 12%-mol), whereas at the same time the content of the 3OHC14:1 monomer decreased (2%-mol).

Cultivation of *Pseudomonas sp.* IPB-B26 in glycerol provided a PHA polymer, having a significantly lower molecular weight distribution but a similar polydispersity index (see Table 4).

**Table 4** Molecular weight distribution of the PHA-polymers produced by strains IBP-B26 and N-128

| Strain/medium-substrate | $M_n$ (kDa) | $M_w$ (kDa) | $M_p$ (kDa) | Dispersity PDI |
|---|---|---|---|---|
| N128/ E2- oleic | 172 | 294 | 241 | 1.7 |
| IPB-B26/ E2- oleic | 145 | 218 | 185 | 1.5 |
| IPB-B26/ E2-glycerol | 56 | 100 | 86 | 1.8 |

Values were determined by GPC (universal calibration): $M_p$ is the molecular weight at peak maximum; $M_n$, molecular weight in number; $M_w$, molecular weight in mass and PDI is polydispersity index.

Further thermal properties of the obtained polymers are presented in the following Table 5.

**Table 5** Thermal properties of the different polymers produced by the strains IPB-B26 and N-128

| Strain/medium-substrate | $T_{g,1}$ (°C) | $\Delta c_{p,1}$ (J g$^{-1}$ K$^{-1}$) | $T_{g,c}$ (°C) | $T_{d,1}$ (°C) | $\Delta H_{d,1}$ (J g$^{-1}$) |
|---|---|---|---|---|---|
| N128/ E2- oleic | -49 | 0.67 | -49 | 297 | 490 |
| IPB-B26/ E2- oleic | -49 | 0.12 | | 297 | 460 |
| IPB-B26/ E2- glycerol | -48 | 0.16 | | 301 | 575 |

$T_g$: glass transition temperature, $T_{g,c}$: cooling run temperature, $\Delta c_p$: change of heat capacity at $T_g$, $T_d$: melting temperature and $\Delta H_d$: melting enthalpy. All obtained from DSC second heating or first cooling run.

Despite of the differences in the monomer composition, all the polymers have similar glass transition temperature (Tg~(-48°C)) and temperature of decomposition around 297-300°C (Table 5), suggesting that the presence of small chain length-monomers with less than 5 carbon atoms is not affecting the thermal behaviour of the polymers.

## Example 4

Batch fermentation of *Pseudomonas sp.* IPB-B26 with oleic acid

*Pseudomonas sp,* IPB-B26 was cultivated in medium E2 using 10 g/L oleic acid as a substrate. The starting stirring was set up at 400 rpm, the temperature at 30°C, the air flow rate at 1 L/min and the p02 (partial oxygen pressure) fixed at 30% and kept using cascade control.

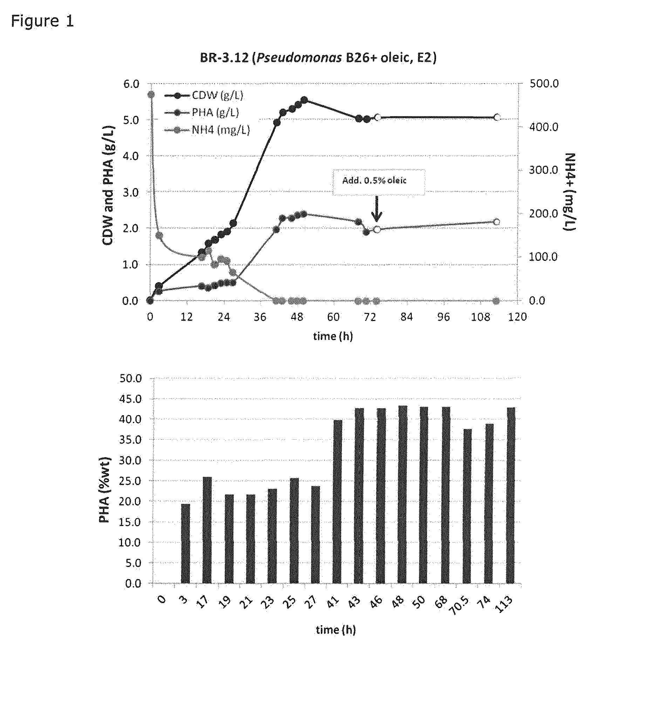

Cell growth started immediately after inoculation. Despite the $pO_2$ declined by 60% within the initial 4 h, the process slowed down and it was not until 30 h of cultivation that the $pO_2$ had to be regulated by the stirring, indicating maximal metabolic activity. Figure 1 shows the kinetic profiles of biomass and PHA production and ammonium consumption along the batch fermentation of *Pseudomonas sp.* IPB-B26 (the values are means of duplicates). According to the growth and PHA production curves, the interval time of cultivation of 30 h to 43 h was the time with the highest rate of biomass and PHA production (see Fig. 1).

After 43 h of cultivation the PHA accumulation reached a maximum of 43 %-wt and then remained almost constant, between 40 and 43 %-wt, over the 110 h of cultivation. No problems of foam formation were detected along the process.

The highest biomass and PHA yields were obtained after 50 h of cultivation, reaching 5.5 g/L and 2.4 g/L, respectively. PHA accumulation was up to 43%-wt (Fig. 1). Ammonium was completely exhausted after 36 h of cultivation, correlating with the highest yield of PHA production although HPLC analysis of the supernatants indicated that the substrate was not fully consumed at the end of fermentation.

After 70 h of cultivation a pulse of 0.5% of oleic acid was added to try to further increase the PHA accumulation, but the substrate was not consumed and no changes in the PHA accumulation were detected.

## Example 5

Fed-batch fermentation of *Pseudomonas* sp. IPB-B26 with oleic acid

The specific growth rate ($\mu$) for strain-substrate and biomass conversion yields ($Y_{x/s}$) are the parameters that need to be calculated for the design of an exponential feeding according the following equation:

$$F(t) = \mu \cdot \frac{Vo \cdot Xo}{So \cdot Yx/s} \cdot e^{-\mu set \cdot t}$$

where F(t) is the flow rate of the carbon source along the cultivation, $V_o$ is the volume of the culture (3 L working volume), $Y_{x/s}$ is the yield of biomass, $X_o$ is the initial biomass after the batch culture and $\mu_{set}$ is the desired specific growth rate.

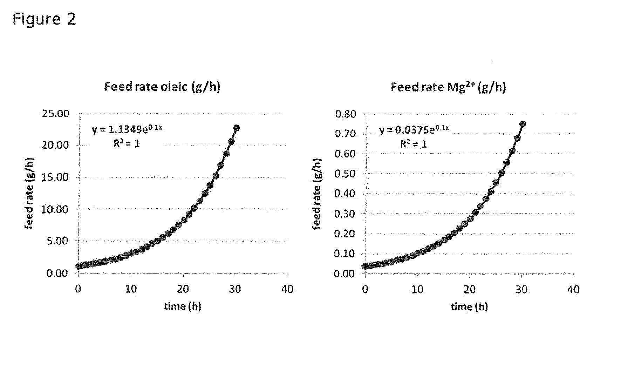

*Pseudomonas sp.* IPB-B26 was cultivated in medium E2 with 3 g/L of oleic acid, using starting stirring of 400 rpm, air flow rate of to 3 L/min, and the $pO_2$ fixed at 30% using cascade control. The kinetic parameters were as follows: $\mu_{set}$ of 0.1 h$^{-1}$, So of 2.67 g/L and $Y_{x/s}$ of 0.89 g/g. The fermentation started with a batch culture with 3.0 g/L of oleic acid during the initial 12 h followed by an exponential feeding during 24 h and a final step consisting of a linear feeding of **1** g/L/h of oleic acid. During the exponential feeding, ammonium was supplied as $NH_4OH$ (14% v/v) using pHstat control. Additional $Mg^{2+}$ was supplied in the ratio of 0.033 g $MgSO_4$/1 g oleic acid (Fig. 2).

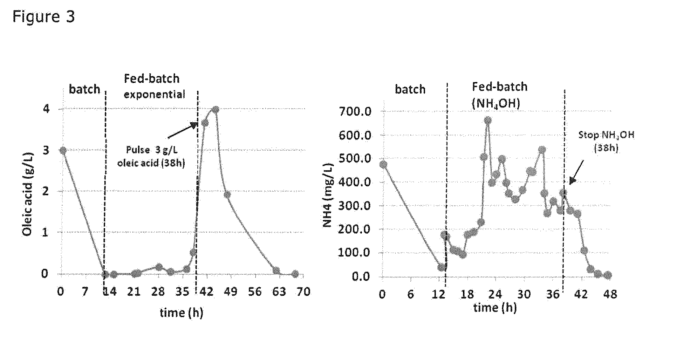

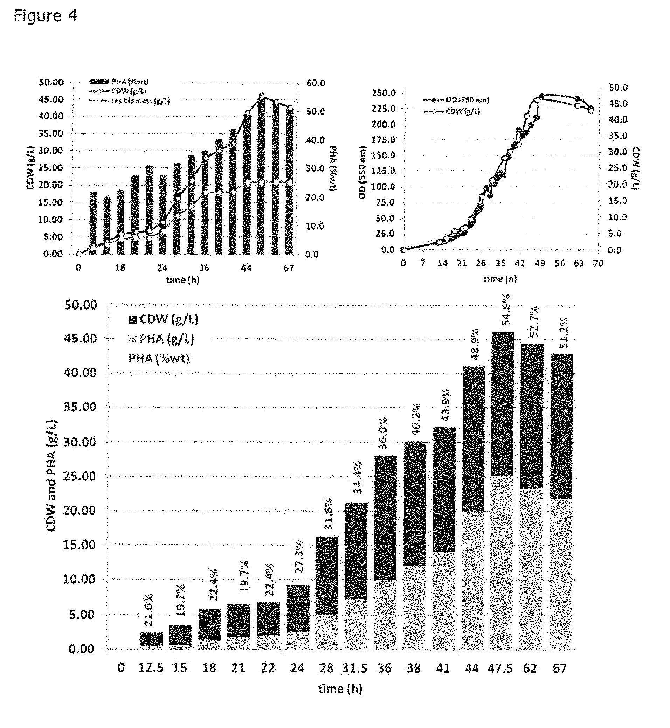

The increase in the stirring indicated that the cells started growing immediately. The carbon source was completely exhausted after the initial 12 h of cultivation, (Fig. 3) and the exponential feeding was carried on for the following 24 h. The stirring speed was kept between 800-1,000 rpm during the whole process, being higher in the phase of maximal growth (from 24 h to 40 h of cultivation). At 38 h of cultivation, the exponential feeding and the ammonium supply was stopped and a pulse of 3 g/L of oleic acid was supplied before starting the 10 h of linear feeding. After the linear feeding, the HPLC analyses showed that the carbon and nitrogen sources were both not fully consumed and therefore the fermentation was carried on until 68 h of cultivation. Both nutrients were completely consumed after 68 h of cultivation (Fig. 3). However, no significant changes in the biomass and polymer production were observed between 44 and 68 h (Table 6 and Fig. 4).

The highest biomass and PHA production yields were achieved after 48 h of fermentation, being of 46.2 g/L and 25.3 g/L, respectively. During the exponential feeding mainly biomass was produced while the highest PHA accumulation took place at the end of the period of the linear feeding, leading to a PHA accumulation of 55%wt, after 48 h of culturing (Table 6 and Fig. 4).

**Table 6** Biomass and PHA production in the fermentation using strain *Pseudomonas sp.* IPB-B26 and oleic acid as substrate

| time (h) | CDW (g/L) | liof-CDW (g/L) | PHA (g/L) | res biomass (g/L) | PHA (%wt) |
|---|---|---|---|---|---|
| 0 | 0.02 | 0.02 | 0.00 | 0.02 | 0.0 |
| 12.5 | 2.41 | 2.91 | 0.52 | 1.89 | 21.6 |
| 15 | 3.50 | 4.02 | 0.69 | 2.81 | 19.7 |
| 18 | 5.81 | 5.73 | 1.30 | 4.51 | 22.4 |
| 21 | 6.48 | 6.82 | 1.77 | 4.71 | 27.3 |
| 22 | 6.79 | 7.73 | 2.09 | 4.70 | 30.8 |
| 24 | 9.33 | 8.97 | 2.55 | 6.78 | 27.3 |
| 28 | 16.29 | 22.04 | 5.15 | 11.14 | 31.6 |
| 31.5 | 21.30 | 22.80 | 7.32 | 13.98 | 34.4 |
| 36 | 28.09 | 27.56 | 10.11 | 17.98 | 36.0 |
| 38 | 30.24 | 30.33 | 12.17 | 18.07 | 40.2 |
| 41 | 32.30 | 33.32 | 14.19 | 18.11 | 43.9 |
| 44 | 41.13 | 41.02 | 20.10 | 21.03 | 48.9 |
| 47.5 | 46.13 | 42.45 | 25.30 | 20.83 | 54.8 |
| 62 | 44.38 | 45.44 | 23.40 | 20.98 | 52.7 |
| 67 | 42.81 | 43.11 | 21.90 | 20.91 | 51.2 |

The lower demand of oxygen during the process, as reflected in the history plot (Fig. 3), is noteworthy particularly considering the high cell density ($OD_{550nm}$ of 250) reached by strain *Pseudomonas sp.* IPB-B26 at the end of the fermentation. In fact, the $pO_2$ was perfectly controlled by the air-flow and the stirring. Foam formation could be controlled by adding 3 mL of anti-foam at the end of the exponential feeding phase.

In the following Table 7, the results from batch- (Example 4) and fed-batch process are compared:

**Table 7** Biomass and PHA yields of the fermentation processes using strain Pseudomonas sp. IPB-B26 and oleic acid as substrate

| Experiment | Batch | Fed-batch |
|---|---|---|
| CDW (g/L) | 5.5 | 46.1 |
| PHA (g/L) | 2.4 | 25.3 |
| PHA (%wt) | 43 | 54.8 |
| time of cultivation (h) | 50 | 47.5 |

*Pseudomonas sp.* IPB-B26 was successfully up-scaled in a 5 L bioreactor using a fed-batch strategy and rendering into biomass and PHA production of 46 g/L and 25.3 g/L, respectively, after 48h of cultivation. These yields represent a 10-fold increase compared to the initial culture strategy, indicating the suitability of the environmental strain Pseudomonas sp. IPB-B26 for PHA production in fermentation processes.

The monomer composition of the obtained polymer was determined by NMR and GC-MS analysis. The polymer was constituted by the following monomer units: C4:0 (0.5 %-mol), C6:0 (5.2 %-mol); C8:0 (38.7 %-mol), C10:0 (29.3 %-mol), C12:0 (14.6 %-mol), C14:0 (0.8 %-mol) and C14:1 (10.9 %-mol). Due to the low content of the C4:0 unit (only 0.5 %-mol) this monomer could not be detected in the NMR analysis, although the presence was confirmed *a posteriori* by the GC-MS analysis. The monomer composition obtained was similar to the previously reported for this strain-substrate combination in the flask experiments.

## Claims

1. A microorganism of the genus *Pseudomonas* as deposited under DSM26199 (*Pseudomonas sp.* IPB-B26) or DSM26200 (*Pseudomonas sp. N-128*) with the DSMZ.

2.  A process for the production of medium- or long-chain-length PHAs, comprising

    - cultivating a microorganism of the genus *Pseudomonas* as deposited under DSM26199 *(Pseudomonas sp. IPB-B26)* or DSM26200 *(Pseudomonas sp. N-128)* with the DSMZ in a suitable medium and in the presence of a carbon source and
    - isolating the PHA from the microorganism.

3.  A process according to claim 2, wherein the medium is E2 medium.

4.  A process according to claim 2 or 3, wherein the carbon source comprises at least one C4 to C20 fatty acid, preferably a C8 to C18 fatty acid, preferably comprising one or more unsaturated moieties.

5.  A process according to any one of claim 2 to 4, wherein the oxygen partial pressure ($pO_2$) is maintained between 25% and 45%, preferably at about 30%, in the cultivating medium (% based on total gas dissolved in the culture medium).

6.  A process according to any one of claim 2 to 5, wherein the nitrogen is present in the culture medium as an ammonium salt, preferably with a molar ammonium concentration in the range of 10 to 50 mM, in particular in the range of 15 to 40 mM.

7.  A process according to any one of claim 2 to 6, wherein the process is a shake-flash- or batch process and the carbon to nitrogen (C/N) ratio in the culture medium is in the range of 9:1 to 70:1, preferably in the range of 15:1 to 50:1.

8.  A process according to any one of claim 7, wherein the carbon source is added as a single lump at the start of the cultivation, preferably in an amount to provide a carbon source concentration in the range of 5 to 60 mM, in particular 10 to 40 mM, in the cultivating medium.

9.  A process according to any one of claim 2 to 6 wherein the carbon source is supplied to the cultivating medium in a fed-batch manner to provide an exponentially increasing carbon source dosage after an initial batch phase, preferably with a specific growth rate $\mu_{set}$ in the range of 0.05 to 0.15, more preferably in the range of 0.08 to 0.12 h$^{-1}$.

10. A process according to claim 9, wherein in the batch phase an initial lump of carbon source, preferably providing a carbon source concentration in the cultivating medium in the range of 2 to 30 mM, more preferably 5 to 15 mM, is added to the cultivation medium and the culture is maintained for a time sufficient to assure complete consumption of the initial carbon source, preferably for 8 to 16 h.

11. A process according to any one of claims 2 to 10, wherein the PHA is isolated by extraction with a ketone having 3 to 8 carbon atoms, preferably with acetone.

12. A process according to claim 2 to 11, wherein the PHA is isolated by extraction at a temperature of 60°C or less, preferably at 20 to 40°C.

13. PHA obtainable by the process of any one of claims 2 to 12, wherein the PHA preferably contains more than 10% of unsaturated moieties.

14. Use of a microorganism according to claim 1 in a process for the production of medium- or long-chain-length PHA.

15. Use of a PHA-synthase as deposited in the Gene Bank (NCBI) under the Accession number JN651420 (phaC1) or JN216885 (phaC2) or analogues thereof or mixtures of these PHA synthases or analogues thereof for the production of PHA, preferably PHA containing carbon-carbon double bonds and/or aromatic moieties.

Figure 1

BR-3.12 (*Pseudomonas* B26+ oleic, E2)

Figure 2

Feed rate oleic (g/h)

$y = 1.1349e^{0.1x}$
$R^2 = 1$

Feed rate Mg$^{2+}$ (g/h)

$y = 0.0375e^{0.1x}$
$R^2 = 1$

Figure 3

Figure 4

Europäisches
Patentamt

European
Patent Office

Office européen
des brevets

# EUROPEAN SEARCH REPORT

Application Number

EP 13 17 3572

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FURRER ET AL: "Efficient recovery of low endotoxin medium-chain-length poly([R]-3-hydroxyalkanoate) from bacterial biomass", JOURNAL OF MICROBIOLOGICAL METHODS, ELSEVIER, AMSTERDAM, NL, vol. 69, no. 1, 28 March 2007 (2007-03-28) , pages 206-213, XP005928196, ISSN: 0167-7012, DOI: 10.1016/J.MIMET.2007.01.002 * abstract * | 1-14 | INV. C12R1/38 C12P7/62 C12N9/10 |
| X | ALEXANDER MUHR ET AL: "Novel Description of mcl-PHA Biosynthesis by Pseudomonas chlororaphis from Animal-Derived Waste", JOURNAL OF BIOTECHNOLOGY, vol. 165, no. 1, 1 May 2013 (2013-05-01), pages 45-51, XP055084933, ISSN: 0168-1656, DOI: 10.1016/j.jbiotec.2013.02.003 * abstract * | 1-14 | |
| | | | TECHNICAL FIELDS SEARCHED (IPC) |
| X | Rosa Palmeri ET AL: "Polyhydroxyalkanoates (PHAs) Production Through Conversion of Glycerol by Selected Strains of Pseudomonas Mediterranea and Pseudomonas Corrugata", chemical engineering transactions, 31 December 2012 (2012-12-31), pages 121-126, XP055084937, Retrieved from the Internet: URL:http://www.aidic.it/cet/12/27/021.pdf [retrieved on 2013-10-22] * abstract * | 1-14 | C12R C12P C12N |

-/--

The present search report has been drawn up for all claims

| Place of search | Date of completion of the search | Examiner |
|---|---|---|
| The Hague | 23 October 2013 | van Voorst, Frank |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

## EUROPEAN SEARCH REPORT

Application Number

EP 13 17 3572

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIN SONG ET AL: "Polyhydroxyalkanoate (PHA) production using waste vegetable oil by Pseudomonas sp. strain DR2.", JOURNAL OF MICROBIOLOGY AND BIOTECHNOLOGY, vol. 18, no. 8, 1 August 2008 (2008-08-01), pages 1408-1415, XP055084938, ISSN: 1017-7825 * abstract * | 1-14 | |
| X | SIMON-COLIN C ET AL: "A novel mcl PHA-producing bacterium, Pseudomonas guezennei sp nov, isolated from a 'kopara' mat located in Rangiroa, an atoll of French Polynesia", JOURNAL OF APPLIED MICROBIOLOGY, OXFORD, GB, vol. 104, no. 2, 1 February 2008 (2008-02-01), pages 581-586, XP002472186, ISSN: 1364-5072, DOI: 10.1111/J.1365-2672.2007.03667.X * abstract * | 1-14 | |
| X | TAEK HO YANG ET AL: "Tailor-made type II Pseudomonas PHA synthases and their use for the biosynthesis of polylactic acid and its copolymer in recombinant Escherichia coli", APPLIED MICROBIOLOGY AND BIOTECHNOLOGY, vol. 90, no. 2, 1 April 2011 (2011-04-01), pages 603-614, XP055051491, ISSN: 0175-7598, DOI: 10.1007/s00253-010-3077-2 * abstract * | 13,15 | TECHNICAL FIELDS SEARCHED (IPC) |

-/--

The present search report has been drawn up for all claims

| Place of search | Date of completion of the search | Examiner |
|---|---|---|
| The Hague | 23 October 2013 | van Voorst, Frank |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**Europäisches Patentamt**
**European Patent Office**
**Office européen des brevets**

# EUROPEAN SEARCH REPORT

**Application Number**

EP 13 17 3572

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIAO-WEN SHEN ET AL: "Engineering of polyhydroxyalkanoate (PHA) synthase PhaC2ofvia site-specific mutation for efficient production of PHA copolymers", APPLIED MICROBIOLOGY AND BIOTECHNOLOGY, SPRINGER, BERLIN, DE, vol. 91, no. 3, 21 April 2011 (2011-04-21), pages 655-665, XP019927349, ISSN: 1432-0614, DOI: 10.1007/S00253-011-3274-7 * abstract * | 13,15 | |
| X | MANFRED SCHMID ET AL: "Autoxidation of Medium Chain Length Polyhydroxyalkanoate", BIOMACROMOLECULES, vol. 8, no. 2, 1 February 2007 (2007-02-01), pages 579-584, XP055084983, ISSN: 1525-7797, DOI: 10.1021/bm060785m * abstract * | 13 | |
| X | DATABASE UniProt [Online]  18 April 2012 (2012-04-18), "SubName: Full=Class II poly(3-hydroxyalkanoic acid) synthase;", XP002715233, retrieved from EBI accession no. UNIPROT:H6UJH7 Database accession no. H6UJH7 * the whole document * | 15 | **TECHNICAL FIELDS SEARCHED (IPC)** |

-/--

The present search report has been drawn up for all claims

| Place of search | Date of completion of the search | Examiner |
|---|---|---|
| The Hague | 23 October 2013 | van Voorst, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.....................................................................

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 13 17 3572

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online]<br><br>6 February 2013 (2013-02-06),<br>"SubName: Full=Poly(3-hydroxyalkanoic acid) synthase class II;",<br>XP002715234,<br>retrieved from EBI accession no.<br>UNIPROT:K7PFD9<br>Database accession no. K7PFD9<br>* the whole document *<br>----- | 15 | |

TECHNICAL FIELDS
SEARCHED        (IPC)

The present search report has been drawn up for all claims

| Place of search | Date of completion of the search | Examiner |
|---|---|---|
| The Hague | 23 October 2013 | van Voorst, Frank |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4876331 A
- US 5292860 A
- EP 1913135 A1

### Non-patent literature cited in the description

- **WILLIAMS ; PEOPLES.** *Chemtech,* 1996, vol. 26, 38-44
- **LEMOIGNE ; ROUKHELMAN.** *Ann. Des Fermentation,* 1925, 527-536
- **DESMET et al.** *J. Bacteriol.,* 1983, vol. 154, 870-878
- **WALLEN ; ROHWEDER.** *Environ. Sci. Technol,* 1974, vol. 8, 576-579
- **STEINBUCHEL ; WIESE.** *Appl. Microbiol. Biotechnol,* 1992, vol. 37, 691-697
- **VALENTIN et al.** *Appl. Microbiol. Biotechnol,* 1992, vol. 36, 507-514
- **VALENTIN et al.** *Appl. Microbiol. Biotechnol.,* 1994, vol. 40, 710-716
- **ABE et al.** *Int. J. Biol. Macromol.,* 1994, vol. 16, 115-119
- **LEE et al.** *Appl. Microbiol. Biotechnol.,* 1995, vol. 42, 901-909
- **KATO et al.** *Appl. Microbiol. Biotechnol,* 1996, vol. 45, 363-370
- **VALENTIN et al.** *Appl. Microbiol. Biotechnol.,* 1996, vol. 46, 261-267
- **BRANDL et al.** *Int. J. Biol. Macromol.,* 1989, vol. 11, 49-55
- **AMOS ; MCINEREY.** *Arch. Microbiol.,* 1991, vol. 155, 103-106
- **HRABAK.** *FEMS Microbiol. Rev.,* 1992, vol. 103, 251-256
- **CHOI ; LEE.** *Bioprocess Eng.,* 1997, vol. 17, 335-342
- **STEINBÜCHEL.** *Biomaterials,* 1991, 123-213
- **LEE.** *Biotech. Bioeng.,* 1996, vol. 49, 1-14
- **QI et al.** *FEMS Microbiol. Lett.,* 2007, vol. 157, 155-162
- **CAI et al.** *Bioresource Techn,* 2009, vol. 100, 2265-2270
- **MARTINEZ-BLANKO et al.** *J. Biol. Chem.,* 1990, vol. 265, 7084-7090
- **CHOI et al.** *Appl. Environ. Microbiol.,* 1994, vol. 60, 1245-1254
- **VOGEL ; BORNER.** *J. Biol. Chem.,* 1956, vol. 218, 97-106
- **CHOI et al.** *Appl. Environ. Microbiol.,* 1994, vol. 60, 3245-3254